# DEMANDE DE BREVET EUROPEEN

(11) **EP 4 477 154 A1**
(43) Date de publication de la demande: **18.12.2024**
(21) Numéro de dépôt: 24180880.7
(22) Date de dépôt: 07.06.2024
(51) Int. Cl.: A61B 10/00, A61B 6/00, A61B 10/02

(54) **SYSTÈMES DE RECUEIL ET D'ANALYSE PEROPÉRATOIRE DE TISSUS BIOLOGIQUES**

(30) Priorité: 09.06.2023 FR 2305857
(71) Demandeur: Beams, 75004 Paris (FR)
(72) Inventeur: BOYER, Bruno, 91000 Palaiseau (FR); MENARD, Laurent, 91120 Palaiseau (FR); PALFI, Stéphane, 91530 Le Val-Saint-Germain (FR); SIMON, Virginie, 41350 Vineui (FR); VILLEDIEU DE TORCY, Estelle, 92220 Bagneux (FR)
(74) Mandataire: IP Trust

(57) **Abrégé**

L'invention présente un système de prélèvement de tissus biologiques dans une zone d'intérêt recevant préalablement ou simultanément à l'intervention chirurgicale un biomarqueur, ledit système comportant une tête d'exérèse reliée à un réceptacle (300) des tissus prélevés par un tuyau de transfert par aspiration, caractérisé en ce qu'il comporte :
- un manchon de détection entourant une zone de référence dudit tuyau de transfert par aspiration, ledit manchon de détection comportant au moins un capteur sensible audit biomarqueur
- un circuit électronique délivrant un signal électrique représentatif de la présence ou de l'absence de marqueur lors du passage d'un tissu prélevé dans ladite zone de référence.

## Description

### Domaine de l'invention

L'invention concerne les équipements chirurgicaux destinés au prélèvement de tous tissus biologiques, en particulier des tumeurs, par une tête de détection et/ou d'exérèse reliée à un tube d'aspiration assurant le transfert des tissus prélevés vers un conteneur recevant les tissus prélevés en vue d'une analyse en temps réel pendant le déplacement dans le tube d'aspiration et/ou d'un diagnostic ultérieur anatomopathologique, incluant les études protéomiques, métabolomiques ou génotypiques.

Afin de faciliter la localisation des tissus à détecter et à analyser, un produit est injecté en per-opératoire soit au voisinage de la zones d'intérêt, soit en intravasculaire ou lymphatique. Trois produits sont le plus souvent utilisés : un colorant (injecté en salle d'opération quelques minutes avant l'intervention chirurgicale), une molécule fluorescente ou un produit radioactif (injecté quelques heures avant l'intervention chirurgicale ou en per-opératoire). Ces produits sont quelques fois associés.

Les tissus prélevés sont détectés, comptés et géolocalisés dans la région tumorale, puis stockés dans des récipients identifiés et labélisés. Les tissus prélevés sont ensuite soit analysés dans le bloc opératoire ou bien envoyés au laboratoire afin d'être analysés extemporanément par une analyse anatomopathologique ou éventuellement par des tests biochimiques et génotypiques.

Ces techniques peuvent également être utilisées pour améliorer la précision du diagnostic tumoral des biopsies en guidant le chirurgien vers des régions tissulaires pertinentes pour déterminer la nature histologique de la tumeur.

Ces techniques permettent de sécuriser le geste opératoire en indiquant en temps réel au chirurgien la nature tumorale ou non des prélèvements tissulaires.

### Etat de la technique

On connaît dans l'état de la technique le brevet US2012283563 qui a pour objet un dispositif de biopsie qui comprend une aiguille, un élément tranchant amovible relativement à l'aiguille pour sectionner un échantillon de tissu, un module de traitement, un détecteur de tissu, et un indicateur. Le détecteur de tissu détecte le passage d'un échantillon de tissu sectionné par l'élément tranchant. L'indicateur émet un signal audible et/ou un indicateur visuel. L'indication fournie par l'indicateur peut varier en fonction des qualités détectées de l'échantillon de tissu. L'indicateur peut être intégré dans un instrument de biopsie ou peut être fourni comme une partie d'une unité distante. Le système de biopsie peut également comprendre un support pour échantillons de tissu à chambres multiples. L'interface utilisateur graphique peut indiquer les chambres du support pour échantillons de tissu qui sont occupées par les échantillons de tissu.

Cette solution permet de vérifier si un échantillon a été aspiré ou non.

Le document US2016100852 décrit un dispositif de coupe de tissu comprenant un arbre central rotatif ayant une première extrémité et une seconde extrémité ; un moteur couplé de manière fonctionnelle à la première extrémité de l'arbre rotatif central, le moteur comprenant des circuits configurés pour faire tourner l'arbre rotatif central ; un composant mobile configuré pour se déplacer le long d'au moins une partie de la longueur de l'arbre rotatif central ; et un composant de coupe flexible allongé ayant une première extrémité et une seconde extrémité, la première extrémité du composant de coupe flexible allongé étant fixée au composant mobile et la seconde extrémité du composant de coupe flexible allongé étant fixée à l'arbre rotatif central à proximité du second extrémité de l'arbre rotatif central ; le mouvement du composant mobile le long d'au moins une partie de la longueur de l'arbre rotatif central modifiant une forme formée par le composant de coupe flexible allongé.

On connaît aussi le brevet US2022370024 décrivant un dispositif de mesures in vivo de produits radiopharmaceutiques utilisés pour le diagnostic et la surveillance de la radiothérapie sont présentés comportant une canule qui peut comprendre une chambre de mesure, un détecteur de rayonnement et une lumière d'administration. Le dispositif est utilisé à la fois pour administrer un matériau au patient (par exemple, des radiotraceurs utilisés dans des produits radiopharmaceutiques) et mesurer les niveaux et les concentrations de matière radioactive dans, par exemple, le sang du patient pendant et après l'administration de la matière radioactive. Dans certains modes de réalisation, les particules émises par le matériau radioactif interagissent avec un matériau scintillant, ce qui entraîne la libération de lumière qui peut être transmise, via le matériau scintillant et/ou le matériau à fibre optique, à un détecteur optique ou à un processeur pour traitement. Dans certains modes de réalisation, des matériaux absorbant les particules peuvent être utilisés pour limiter les mesures aux matériaux situés à l'intérieur de la chambre de mesure ou dans une autre zone d'intérêt.

On connaît aussi dans l'état de la technique le brevet FR2908283 qui a pour objet un dispositif doté d'une partie insérable dans une cavité tubulaire comportant : une sonde miniaturisée de détection de rayonnements gamma comportant une partie insérable dans une cavité tubulaire qui comprend au moins : un cristal scintillateur qui émet de la lumière en réponse à des rayonnements ionisants, de préférence à des rayonnements gamma, et qui présente un volume inférieur à 30 mm3, la section droite transversale maximale de la sonde étant inférieure à 10 mm2, une fibre optique assurant la collecte d'au moins une partie de la lumière émise par le matériau scintillateur, et sa transmission vers un convertisseur photoélectrique, un convertisseur photoélectrique auquel est relié le cristal par l'intermédiaire de la fibre optique qui assure la collecte et la transmission d'au moins une partie de la lumière émise par le cristal scintillateur vers le convertisseur photoélectrique, et des moyens optiques de visualisation de la cavité, insérable dans cette dernière.

La demande de brevet US2017131311 décrit un système d'analyse d'échantillons de biopsie qui comprend un mécanisme de transport d'échantillon de tissu dépendant d'un outil d'excision d'échantillon de biopsie à un support d'échantillon de tissu disposé dans une zone de présentation. d'une unité d'analyse. L'échantillon de tissu est automatiquement transporté de l'outil d'excision au support d'échantillon, l'échantillon de tissu étant analysé dans la zone de présentation de l'unité d'analyse. Le mécanisme de transport peut comprendre une tubulure et une source de vide. Le support d'échantillon de tissu peut être configuré pour ralentir ou arrêter temporairement un échantillon de tissu pour une analyse individuelle, ou pour prélever de multiples échantillons à analyser comme un groupe. Un mécanisme de tri d'échantillons de tissu peut être utilisé, qui permet la séparation des échantillons qui peuvent être corrélés à l'analyse.

La demande de brevet WO2018102713 décrit un dispositif de biopsie comprenant un corps, une aiguille, un instrument tranchant, un porte-échantillon tissulaire et un ensemble porte. L'aiguille s'étend de manière distale dans le prolongement du corps. L'instrument tranchant est longitudinalement mobile par translation par rapport à l'aiguille et définit une lumière d'instrument tranchant. Le porte-échantillon tissulaire est accouplé de manière proximale par rapport au corps. La lumière d'instrument tranchant de l'instrument tranchant délimite au moins une partie d'une conduite de fluide s'étendant entre l'instrument tranchant et le porte-échantillon tissulaire. L'ensemble porte est conçu pour arrêter sélectivement un déplacement d'un porte-échantillon tissulaire à l'intérieur de la conduite de fluide entre l'instrument tranchant et le porte-échantillon tissulaire.

### Inconvénient de l'art antérieur

Les solutions de l'art antérieur ne sont pas totalement satisfaisantes car le réceptacle de collecte contient après l'intervention un mélange de tous les tissus prélevés, dont des tissus sains qui compliquent le travail ultérieur d'analyse et empêche de se concentrer sur l'étude des tissus pathologiques. Par ailleurs la gestion des déchets par les hôpitaux des produits radioactifs est très réglementée. En effet, certaines pathologies, telles que les pathologies neurologiques, comporte un risque et implique que tous les déchets utilisés pour le traitement soient détruits : les déchets sont alors tracés, stockés puis envoyés dans une zone de déchets identifiée. Le mélange de tissus marqués et de tissus non marqués augmente le volume des déchets spéciaux nécessitant un traitement coûteux pour leur élimination.

Les solutions de l'art antérieur permettent de contrôler quantitativement en temps réel la pertinence de l'exérèse tumorale de tissus détecté par le marqueur fluorescent ou radioactif mais pas de corréler des données de comptage à d'autres informations pour une détection plus sensible des traceurs en isolant la détection du « bruit » de la plaie opératoire.

Les solutions proposées par les brevets US2012283563 ou US2016100852 n'apportent pas de solution car elles permettent de détecter le passage d'un matériau biologique, mais pas de discerner s'il s'agit d'un tissu cancéreux ou non.

### Description détaillée d'un exemple non limitatif de réalisation

La présente invention sera mieux comprise à la lecture de la description qui suit, concernant un exemple non limitatif de réalisation illustré par les dessins annexés où :
[FIG. 1] la figure 1 représente une vue schématique d'un système de prélèvement selon l'invention
[FIG. 2] la figure 2 représente une vue schématique d'une variante de réalisation du système de prélèvement selon l'invention

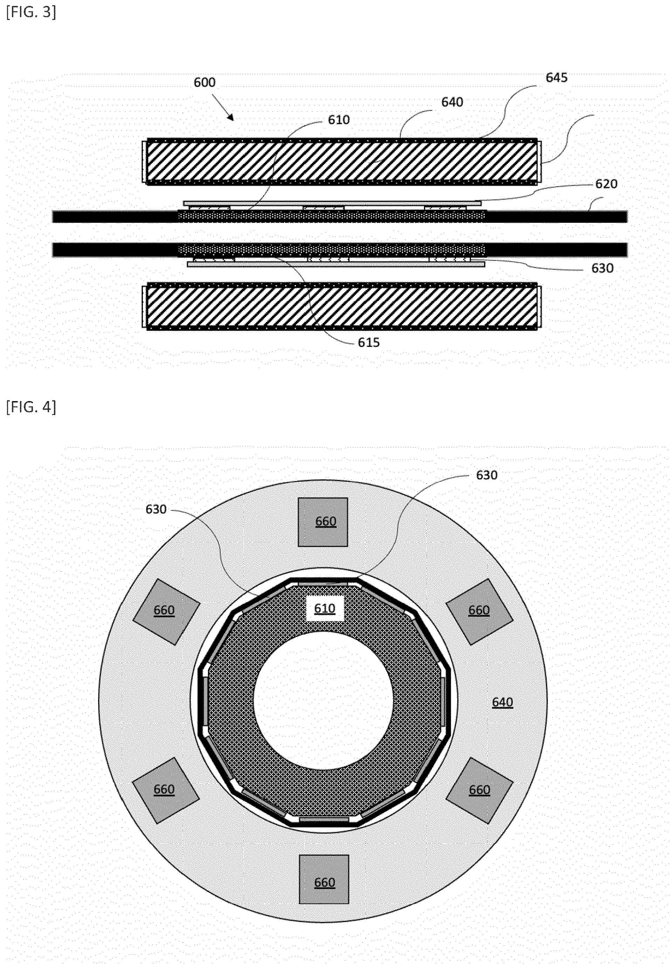

[FIG. 3] la figure 3 représente une vue schématique en coupe longitudinale d'un manchon de détection pour un système de prélèvement selon l'invention
[FIG. 4] la figure 4 représente une vue schématique en coupe transversale d'un manchon de détection pour un système de prélèvement selon l'invention

### Principe général de l'invention

Le système selon l'invention est constitué par :
- Un outil d'exérèse (100)
- Un tuyau d'aspiration (200) préférentiellement à usage unique
- Un ensemble de réceptacles (300)
- Une source de vide (400) éventuellement raccordée par un tube de raccordement (325) à la source de vide de l'hôpital
- Un terminal informatique (500)
- Un manchon de détection (600) entourant le tuyau d'aspiration (200)

La description qui suit précise les caractéristiques d'exemple de réalisation de certains des composants du système. Il est précisé que chacun des composants peut être développé et utilisé indépendamment des autres et qu'il convient de ne pas considérer qu'ils sont nécessairement tous combinés entre eux.

Le principe général de l'invention consiste à prévoir sur le tube d'aspiration (200) reliant la tête d'exérèse (100) aux réceptacles (300) des produits aspirés un ou plusieurs détecteurs destinés à caractériser en temps réel, pendant l'aspiration, dans le tuyau d'aspiration (200), la présence et/ou la nature de l'échantillon prélevé traversant le tuyau, afin de commander une ou plusieurs fonctions telles que l'aiguillage vers un réceptacle particulier de l'ensemble (300) ou l'émission d'un signal sonore ou lumineux en cas d'aspiration excessive de prélèvements non marqués.

### Outil d'exérèse et tête de détection

L'invention n'est pas limitée à un type d'outil d'exérèse particulier, ni à une solution unique d'aspiration douce, mais peut être mise en oeuvre avec de tout type d'outil exérèse ou d'aspiration douce faisant transiter les prélèvements dans un tuyau.

Selon une variante de réalisation, l'outil d'exérèse (100) est constitué d'une tête (110) (de préférence à usage unique) formée par un élément tubulaire traversé par un canal d'aspiration présentant plusieurs géométries possibles, modifiables en fonction de la cavité opératoire, de la sensibilité ou de la résolution nécessaire.

Cette tête (110) est complétée par un système de photo-détection (120), couplé lui-même à un terminal informatique (500) piloté par un logiciel de contrôle, d'acquisition et de visualisation des données.

La tête de détection (110) permet de mesurer la concentration en particules β dans les tissus au voisinage de l'embouchure d'aspiration. Le signal ainsi reçu est transformé par le système de photodétection en un signal électrique ; ce signal électrique est transmis au module d'acquisition de données. Les données sont acquises, traitées et visualisées grâce à un logiciel informatique sur un terminal (500).

La tête de détection et d'exérèse est raccordée à une fibre optique équipée à l'une de ses extrémités d'un cristal scintillateur. Elle peut être insérée dans la cavité tubulaire et déplacer au sein de cette dernière, afin de détecter la présence de toute source de rayonnements gamma, correspondant à un tissu potentiellement cancéreux ayant réagi avec le biomarqueur. L'autre extrémité de la fibre optique est connectée à un convertisseur photoélectrique. Le plus souvent ce convertisseur photoélectrique. La fibre optique constitue un moyen de collecte et de transmission de la lumière émise par le cristal. La fibre optique peut présenter un coeur inorganique, par exemple de silice, ou polymère, par exemple de PMMA. La partie de la lumière émise par le cristal qui est collectée par la fibre optique est transmise par cette dernière au convertisseur photoélectrique qui va assurer sa conversion en un signal électrique et notamment en pulses électriques. De façon avantageuse, le couplage entre la fibre optique et le cristal assure la collecte de plus de 5% des photons de scintillation émis par évènement, et de préférence de 5 plus de 10%. Pour optimiser la lumière collectée, la fibre optique est, de préférence, connectée à une face du cristal selon une section de connexion et le rapport entre la surface du cristal sur laquelle la fibre est connectée et la surface de la section de connexion est de préférence proche de 1 et de préférence inférieur ou égal à 5. 10 La fibre optique est souple et va donc pouvoir circuler et s'adapter à la forme de la cavité dans laquelle elle va être insérée. Il est possible, en fonction de l'application, d'utiliser une fibre de très grande longueur, et en particulier d'une longueur supérieure ou égale à 10 m. De façon avantageuse, le coeur de la fibre présente un diamètre de préférence inférieur à 1,5 mm et la fibre totale (coeur + 15 gainage) présente un diamètre de préférence inférieur à 3 mm. Selon un mode de réalisation préférée, la totalité de la partie insérable peut être courbée avec un rayon de courbure inférieur à 5 cm, de préférence inférieur à 2 cm. Pour obtenir un rayon de courbure inférieur à 1,5 cm avec des fibres à coeur de silice, plusieurs fibres pourront être couplées au même cristal. De façon avantageuse, la partie insérable présente une section (prise transversalement à l'axe longitudinal de la sonde) maximale comprise entre 0,1 et 10 mm2 et de préférence comprise entre 0,2 et 5 mm2. Selon une variante du dispositif, celui-ci comprend également des moyens de quantification des rayonnements gamma reçus par le cristal, par analyse des signaux électriques émis par le convertisseur photoélectrique. Cette analyse peut, par exemple être réalisée par une unité de comptage. Le taux de comptage reflète alors la quantité de rayonnements gamma directement liée à la distance entre la zone radioactive d'émission et la sonde. Le dispositif peut également intégrer des moyens de calcul de la distance entre la sonde et la zone radioactive détectée. La lumière transmise peut être convertie, avant analyse, par un photomultiplicateur ou une photodiode. Selon un mode de réalisation, le signal issu du convertisseur photoélectrique (qui peut être amplifié si nécessaire) est connecté à un discriminateur (SCA pour Single Channel Analyser). Le signal issu du discriminateur est ensuite envoyé sur une échelle de comptage. Dans le cas de l'utilisation de deux cristaux un lent et un rapide, il est possible de dupliquer le signal issu du convertisseur photoélectrique et de le rediriger vers deux discriminateurs. L'un des discriminateurs aura un seuil réglé pour la détection de photons émis par un cristal rapide pour donner suite à l'absorption de rayonnement gamma, l'autre aura un seuil réglé pour la détection de photons issus de la détection des bêta, ces derniers arrivant dilués dans le temps dans le cas d'un scintillateur lent. La sonde selon l'invention peut être utilisée pour la détection de rayonnements gamma issus d'un des isotopes choisi parmi : 99Tc, 1231, 18F ou tout autre isotope émettant des photons gamma d'une énergie supérieure à 50 KeV. La sonde selon l'invention est également adaptée pour la détection de rayonnements ionisants de haute énergie, en particulier de rayonnements gamma d'énergie supérieure à 300 KeV. La sonde selon l'invention, du fait de sa miniaturisation, va pouvoir être intégrée ou insérée en tant qu'accessoire dans différents dispositifs médicaux ou de diagnostic tels que les cathéters, et notamment les cathéters multicanaux, les endoscopes, les instruments de célioscopie, des dispositifs utilisés pendant des actes chirurgicaux. Un endoscope comporte traditionnellement des moyens optiques de visualisation de la cavité dans laquelle il est inséré, ainsi qu'un canal opérateur et/ou un canal d'introduction. Les moyens de visualisation peuvent être une ou plusieurs fibres optiques reliées à un convertisseur optoélectronique permettant d'obtenir une image de la cavité. Il est également possible d'utiliser une caméra miniaturisée qui va être positionnée à l'extrémité distale de l'endoscope. La très faible associée à la forme souple de la fibre optique assurant la déportation du signal émis par le cristal scintillateur permet une utilisation directe de la sonde selon l'invention dans les canaux endoscopiques standard. Les moyens optiques de visualisation et la sonde de détection de rayonnements gamma sont positionnés dans un tube de guidage éventuellement compartimenté. La sonde peut, par exemple, être introduite directement dans le tube d'introduction ou le canal opérateur de l'endoscope et le cristal scintillant positionné à l'extrémité distale de l'endoscope. Le couplage de la technique traditionnelle de détection optique avec la sonde selon l'invention est particulièrement avantageux. Tout d'abord, la sonde permet de détecter les isotopes radioactifs qui s'accumulent dans les tumeurs et permet ainsi de conforter le diagnostic optique pour les tumeurs visibles. De plus, la sonde selon l'invention permet de détecter des tumeurs présentes en profondeur des tissus et donc invisibles par observation directe. La détection d'un marquage par rayonnement ionisant, grâce à la sonde selon l'invention, offre une complémentarité importante aux systèmes endoscopiques optiques existants : détection de lésions de petites tailles, détection de zones non superficielles (1 à 5 cm au-delà des parois et non accessible en optique) et plus généralement la détection dans des zones fortement contrariées où l'analyse optique reste délicate, ... Cette technique présente un très grand potentiel et un champ d'applications médicales très important, qui pourra évoluer et s'enrichir avec le développement de sondes et/ou radiopharmaceutiques de plus en plus spécifiques. Les exemples ci-après permettent d'illustrer l'invention. 25 Exemple 1 : Le détecteur est un cristal rectangulaire de LYSO : Ce3+ (Fibercryst SAS, France à 0,5 % de Ce) de dimensions 3*1*1 mm poli sur toutes ses faces. La fibre optique est une fibre Thorlabs de référence BFH37-800 de coeur silice 800 mm et une ouverture numérique de 0,37. Le diamètre total de la fibre est de 1,4 mm. La fibre mesure 1 m et est polie aux deux extrémités. Le cristal est collé sur une face de la fibre par une colle époxy à deux composants (Géofix de chez ESCIL) transparente à la lumière émise par le cristal. La tête de la sonde est ensuite peinte avec une peinture acrylique blanche, puis l'ensemble de la sonde est peint avec peinture acrylique noire. L'extrémité de sortie de la sonde est placée sur un photomultiplicateur Hamamatsu R647. Le signal du photomultiplicateur est envoyé à un préamplificateur puis un amplificateur de mise en forme, puis il est récupéré sur un compteur d'impulsions également nommés coups. La sonde est ensuite placée au contact d'une source radioactive de 137Cs (émetteur gamma 662 KeV) d'activité 20 KBq. La géométrie de la source peut être assimilée à un disque de 3 mm de diamètre. La sonde est ensuite reculée parallèlement à l'axe du disque par pas de 0,25 mm, le nombre de coups mesurées est intégré sur une période d'une seconde.

### Structure de la tête de détection

La tête de détection (120) est composée d'un assemblage d'éléments de détection organisés circulairement autour d'un outil d'exérèse (110). La lumière de scintillation issue des éléments de détection fibrés est directement collectée et détectée par un système de photodétection (bague de détection), constitué de photomultiplicateurs silicium. Les signaux électriques produits par chaque SiPM sont acheminés vers un système électronique d'acquisition qui quantifie le spectre et taux de comptage des particules beta détectés sur chaque élément fibré.

Chacun des éléments de détection de la tête est composé de deux parties : une fibre optique claire, surmontée d'une fibre optique scintillante. La fibre optique scintillante permet de détecter les émissions radioactives (y ou β). Lorsqu'une particule γ ou β interagit avec la fibre optique scintillante, une gerbe de photos de scintillation est créée. La fibre optique claire sert alors de guide de lumière, dirigeant les photons de scintillation en-dehors de la plaie opératoire.

Après son parcours dans les tissus, une particule β+ peut s'annihiler en rencontrant un électron. Une telle annihilation produit deux photons γ de 511keV qui, s'ils sont détectés, viennent perturber la mesure directe des particules beta. En effet, du fait leur important parcours moyen, les photons détectés peuvent provenir de zones d'accumulation non-spécifiques du radiotraceurs, situées à distance de la région d'intérêt. Il convient donc de rejeter ce bruit de fond pour assurer la fiabilité de la mesure.

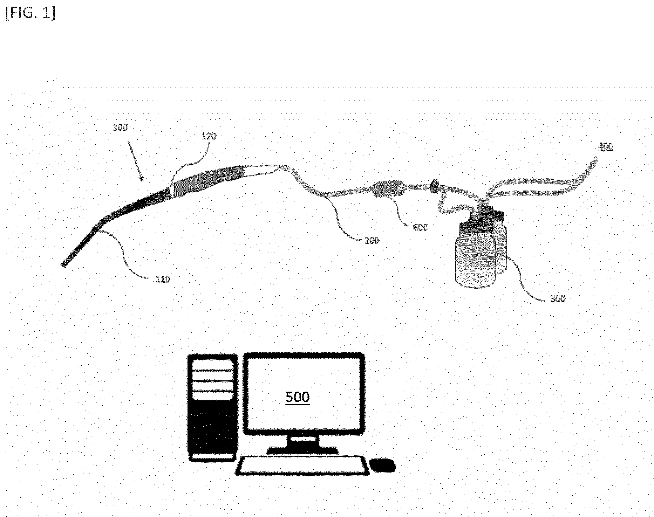

Pour ce faire, le flux de photons atteignant la sonde est évalué en plaçant des éléments de détection sensibles uniquement aux rayonnements γ. Ces éléments de détection sont des fibres optiques recouvertes d'un blindage en acier inoxydable, appelées fibres « témoin », par opposition aux fibres non blindées, appelées fibres « signal » (Figure 1). Le signal issu des fibres témoin est soustrait à celui issu des fibres signal, pour ne conserver que le signal β pur.

L'épaisseur des parties scintillantes des fibres « signal » est optimisée pour atteindre le meilleur compromis en termes de dépôt d'énergie maximal des positrons et de contamination minimale du bruit de fond gamma. La longueur de la partie scintillante des fibres « témoin » est définie de manière à réduire les fluctuations statistiques dans la mesure du signal gamma tout en minimisant la dépendance de la méthode de soustraction par rapport aux variations spatiales du bruit de fond.

Pour optimiser la collecte de lumière par chaque élément de détection, il a été choisi d'ajouter un revêtement optique sur chacune des fibres, en les recouvrant d'une fine couche de peinture réfléchissante qui rétro-guide, à travers la fibre claire, les photons de scintillations émis dans la direction opposée. Ce revêtement permet d'améliorer la sensibilité de la sonde.

Le choix d'utiliser des fibres optiques comme élément de détection pour la sonde s'explique par le fait que celles-ci sont à la fois adaptées à la détection des positons, tout en étant peu sensibles au bruit de fond γ.

Par ailleurs, les fibres pouvant avoir des formes et des tailles variées, permettent d'avoir de la souplesse pour concevoir la géométrie de la tête de détection. De la sorte, en fonction du contexte clinique, de la pathologie traitée, de la forme de la cavité opératoire, de l'outil d'exérèse choisi, etc. plusieurs formes de tête pourront être proposées.

D'un point de vue clinique, les fibres optiques sont également garantes de la sécurité du patient et du chirurgien : elles sont inertes d'un point de vue électrique, ce qui permet leur introduction dans la plaie opératoire et le couplage de la sonde avec un outil d'exérèse. Elles sont enfin compactes, ce qui permet de concevoir une sonde miniaturisée.

Le choix d'une tête de détection à usage unique simplifie les interventions postopératoires de stérilisation de l'outil, inhérentes à la pratique de la chirurgie, et permet au dispositif de conserver une bonne sensibilité. En effet, une approche alternative consisterait à protéger la sonde par une housse en plastique. Or l'ajout d'une telle housse viendrait réduire les performances de la tête de détection.

La tête de détection est composée de fibres optiques en plastique et d'une partie métallique. La partie métallique pourrait a priori être récupérée et réutilisée pour la même utilisation. Les fibres optiques étant en plastique, une étude pourrait être menée sur l'opportunité de les recycler.

Le principe d'utilisation d'un tel outil d'exérèse est le suivant : après avoir retiré la partie principale visible de la tumeur préalablement radiomarquée, le chirurgien introduit la sonde dans la plaie opératoire. Il retire d'abord la partie visible de la tumeur à l'aide d'un outil d'exérèse, par exemple un aspirateur ultrasonore.

On procède ensuite à l'injection par voie intraveineuse d'un radiotraceur émetteur β pendant l'opération, par voie intraveineuse, 30 à 60 minutes avant la vérification des marges tumorales afin de minimiser les contacts radioactifs avec le personnel du bloc opératoire.

On mesure ensuite la concentration en particules β à l'aide de la tête de détection pour localiser les résidus tumoraux et procéder à leur retrait et aspiration.

La mesure locale de la concentration du traceur radioactif autour de l'outil d'exérèse permet de localiser d'éventuels résidus tumoraux. La sonde à détecteur est capable d'accélérer le processus de recherche des tissus cancéreux et de renforcer la qualité du geste opératoire en permettant au chirurgien de définir plus précisément et en temps réel les marges de la résection tumorale, ce qui offre une amélioration du confort et de la sécurité.

Les chirurgiens peuvent donc retirer plus précisément les tissus tumoraux tout en préservant les tissus sains alentours, permettant de davantage préserver les organes touchés. Cette chirurgie, moins lourde et plus conservative, permet au patient de se rétablir plus rapidement et de conserver une meilleure qualité de vie, en limitant le nombre de récidives.

Lorsqu'il utilise une tête de détection (120), le chirurgien pourra choisir entre deux modes de visualisation pour recevoir une information en temps réel sur la localisation des tissus tumoraux : un mode « compteur » et un mode « imageur ». Le mode compteur fournit le taux de comptage β dans l'ensemble des fibres optiques dont est composée la tête de détection. La valeur numérique est affichée sur l'écran du terminal (500) et est complétée par un signal sonore ayant une fréquence proportionnelle au taux de comptage ce qui permet de réaliser l'exploration sans quitter des yeux la plaie opératoire. En mode imageur, chaque fibre optique est représentée visuellement sur l'écran ; le taux de comptage pour chacune des fibres est spécifié, accompagné d'une couleur dont l'intensité est proportionnelle à la valeur indiquée. Cette image pourra être présentée sur un écran dédié ou directement sur le microscope utilisé par le chirurgien, par exemple.

### Caractérisation en ligne des tissus prélevés traversant la zone de référence

L'invention se distingue de l'état de la technique plus particulièrement par la détection dans le flux traversant le tube d'aspiration (200) du passage de prélèvements marqués avant le transfert dans un réceptacle (300). Cette détection est réalisée par un manchon de détection (600) disposé au niveau d'une zone de référence du tube d'aspiration, à une distance connue par rapport au débouché dans le réceptacle (300).

La caractérisation des tissus, et notamment la présence ou l'absence d'un marqueur, est effectué par un manchon (600) comprenant un ou plusieurs capteurs adaptés au mode de traçabilité prévu pour le système d'exérèse.

Ce manchon (600) de détection traversant le tube d'aspiration (200) est disposé sur une zone de référence du tube (200), déterminé par une distance D₁ par rapport à l'extrémité aval du tube (200) et une distance D₂ de l'extrémité amont du tube (200). La détection peut être effectuée par des barrières optiques simples ou équipées de caméras, ou encore par des compteurs de rayonnement ou de particules.

La connaissance de la distance D₁ et de la vitesse moyenne de déplacement des prélèvements dans le tube d'aspiration permet de déterminer le décalage temporel du passage dans une vanne d'orientation (230) dirigeant les prélèvements marqués vers un premier réceptacle (310) par l'intermédiaire d'un embranchement (315), ou vers un réceptacle de déchets (320) via un autre embranchement (325).

Les deux réceptacles sont reliés à la source de dépression (400).

Les informations provenant du manchon (600) permettent de piloter la vanne (230) en fonction de la détection ou non d'un marqueur.

### Traitement des données.

Le manchon (600) comporte un ou plusieurs capteurs de détection du passage d'une matière, ou de détection du passage d'un échantillon marqué. Ces capteurs comportent un microcontrôleur pour exploiter les signaux électriques générés par la détection. Ces signaux font ensuite l'objet d'un traitement comprenant toute ou partie des fonctions suivantes :
- Échantillonnage et codage des signaux pour fournir une trame de données numériques
- Horodatage de la trame de données ou des données
- Filtrage des données pour ne transmettre que les données d'intérêts
- Ajout d'un identifiant incrémental aux données

Ces données sont ensuite exploitées par une application par le terminal (500) pour fournir des fonctionnalités telles que :
- L'aiguillage de l'échantillon vers l'un des réceptacles (300) en fonction de la caractérisation de l'échantillon considéré,
- Le comptage du nombre d'échantillons marqués
- La corrélation avec d'autres information telles que la pression d'aspiration ou l'activation de l'aspiration, ou encore l'image acquise par la tête de détection avec le cas échéant un décalage temporel
- L'activation d'un signal sonore ou lumineux.

### Réceptacles

Alternativement l'ensemble de réceptacle (300) comprend un barillet formé de plusieurs réceptacles déplacés après chaque transfert d'un échantillon. Les informations fournies par le manchon (600) sont dans ce cas horodatées et enregistrées dans une table en relation avec le réceptacle dans lequel l'échantillon, caractérisé lors du passage dans la zone de référence, a été transféré.

Les réceptacles comportent selon une variante une balance de précision PBS fournissant en temps réel une information sur la masse de prélèvements collectés.

Les réceptacles peuvent être équipés de capteurs individuels de radioactivité.

Ils peuvent également être réfrigérés pour améliorer la conservation des tissus prélevés ou contenir une solution de fixation des tissus prélevés tels que du formaldéhyde.

### Radioprotection de l'ensemble de réceptacles

Selon un mode de réalisation préféré, les réceptacles (310, 320) sont enfermés dans une enceinte confinée de radioprotection. Cette enceinte confinée présente avantageusement un espace pour loger la sonde urinaire (450 du patient, et ainsi confiné toutes les sources de rayonnement du patient dans une enceinte de radioprotection (800).

L'enceinte est constituée par un caisson dont les parois comportent des feuilles de plomb, typiquement des feuilles d'une épaisseur totale adaptée à l'absorption de rayonnements gamma d'énergie inférieur à 1 MeV, destinées à confiner les radiations gamma émises par les prélèvements ainsi que par l'urine du patient. Le caisson présente un logement pour recevoir une poche à urine, ainsi qu'un logement pour recevoir l'ensemble de réceptacles de prélèvements (300). Des passages sont prévus pour le passage des conduits de la poche à urine et le tube (200) de transfert des prélèvements, ainsi que le tube de raccordement à la source de vide de l'hôpital. Alternativement, certains passages sont remplacés par des connecteurs fluidiques.

Le réceptacle (310) sécurisé aux rayonnements présentera préférentiellement des compartiments séparés équipés chacun avec un capteur de rayonnement (360) pour recevoir d'une part les échantillonnés sélectionnés, les échantillons rejetés et le cas échéant des récipients de fluides du patient. Le réceptacle accueillera de préférence les échantillons sélectionnés, la collection de tissu rejetés (avec un capteur spécifique) et la poche à urine avec un capteur (350) et une indication de volume (la poche à urine ne sera pas visible à travers le confinement blindé et il est important pour le chirurgien et l'anesthésiste de connaître le volume, la couleur (présence de sang par exemple), concentration etc...).

### Capteurs

Différents types de capteurs peuvent être utilisés pour la caractérisation de l'échantillon, notamment une barrière lumineuse formée par une source lumineuse orientée transversalement par rapport à l'axe du tube (200) associée à un photodétecteur ou une barrette de photodétecteurs ou encore une caméra pour le comptage des échantillons et éventuellement pour une imagerie de la section de l'échantillon et/ou un photodétecteur détectant la luminescence d'un échantillon marqué par une marqueur phosphorescent, ou encore un photodétecteur associé à une source d'excitation détectant la luminescence d'un échantillon marqué par une marqueur fluorescent, ou encore un détecteur capable de compter la radioactivité d'un échantillon marqué par un traceur radioactif.

La vanne (230) peut être commandée automatiquement à partir des signaux fournis par le manchon (600), ou manuellement par le chirurgien ; dans ce dernier cas, le manchon (600) commande l'activation d'un signal sonore et/ou lumineux lors de la détection d'un échantillon marqué, et le chirurgien décide lors de la perception de ce signal si l'échantillon considérée doit être conservé ou rejeté, en tenant compte éventuellement d'autres critères provenant de l'intervention en cours.

Le manchon (600) peut également être raccordé à un spectromètre de masse pour caractériser la présence ou l'absence de certaines protéines ou de traceurs exogènes (tels que le glucose, la dopamine, ...) ou encore une signature biochimique d'une substance d'intérêt.

### Capteur sensible à la radioactivité

Selon un mode de mise en oeuvre préféré, un capteur compte la radioactivité des échantillons aspirés. A cet effet la zone de référence du manchon (600) comprend un segment tubulaire (610) intérieur constitué par un scintillateur dédié à la détection des particules bêta. Ce segment tubulaire (610) intérieur est entourés de photodétecteurs (630) câblés sur un circuit imprimé (620) et recouvert d'un revêtement réfléchissant (615) (sauf sur les zones où sont couplés les photodétecteurs).

Ce premier scintillateur (610) est entouré par un second segment tubulaire (640), coaxial avec le premier, et constitué par un scintillateur dédié à la détection des rayonnements gamma. Il est recouvert par un revêtement réfléchissant (645) et équipés de photodétecteurs (660) câblés sur un circuit imprimé.

La zone de détection est ainsi constituée de deux scintillateurs (610, 640) concentriques. Des photodétecteurs (de type Silicium PhotoMultiplier ou SiPM) sont couplés aux scintillateurs, sur leur face externe et/ou à leur extrémité.

Le scintillateur interne, dit « scintillateur bêta » (610) est un scintillateur organique annulaire qui détecte les particules bêta et les rayonnements gamma. Il est cylindrique sur sa face interne et facetté sur sa face externe pour un contact optimal avec les photomultiplicateurs silicium (630) chargés de recueillir le signal.

Le scintillateur externe, appelé « scintillateur gamma » (640), est un scintillateur inorganique également annulaire, plus dense que le scintillateur interne, (610) qui est superposé à celui-ci et qui ne détecte que les rayons gamma - le faible parcours moyen des particules bêta émis par l'échantillon ne leur permet pas d'atteindre le scintillateur gamma (640).

Grâce aux données collectées par les deux scintillateurs (610, 640) par l'intermédiaire des photodétecteurs (630, 660), il est possible de déduire le taux de comptage beta ou gamma de l'échantillon proportionnel à la quantité de traceur radioactif présent dans l'échantillon, en utilisant une méthode de coïncidence
Le système permet ainsi de mesurer :
- Le taux de comptage beta (avec correction du signal gamma) ;
- Le taux de comptage gamma seul.

Une alerte de sécurité visuelle ou sonore est déclenchée lorsqu'un échantillon traverse le manchon de détection sans provoquer de détection de particules beta. Dans ce cas, l'échantillon a été prélevé dans une zone non-marquée et donc non cancéreuse, et l'opérateur est prévenue par l'alerte afin qu'il puisse éviter de poursuivre des prélèvements inutiles.

### Capteurs additionnels

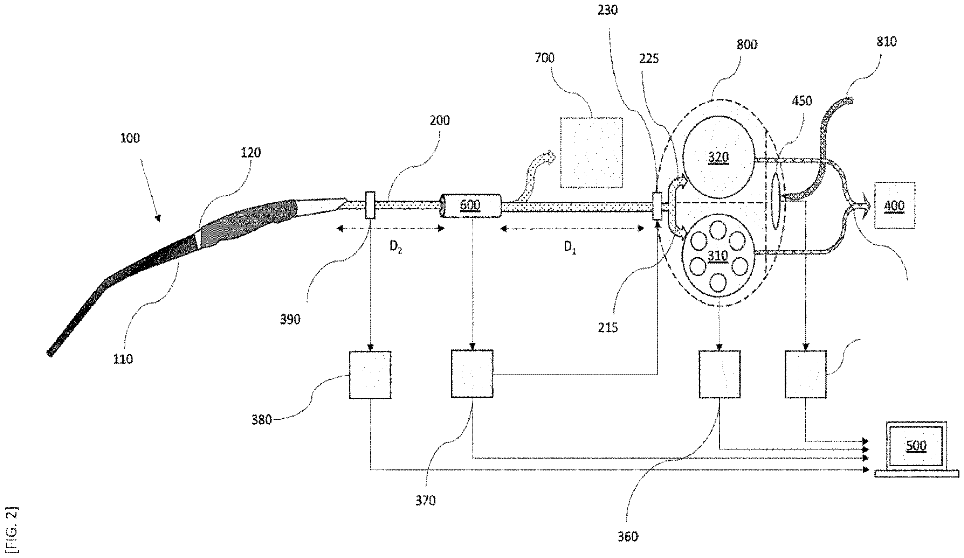

La figure 2 représente un exemple de réalisation comportant des capteurs additionnels.

Une barrière lumineuse (390) formée par une source lumineuse disposés pour former un faisceau traversant transversalement le tuyau (200) et un photodétecteur délivre un signal variant lors du passage d'un prélèvement dans le champ du faisceau de la barrière lumineuse (390). Un microcontrôleur (380) réalise un traitement du signal électrique délivré par la barrière lumineuse (390) pour délivrer une information horodatée du passage de l'échantillon, ou avec un identifiant corrélé avec un identifiant ou un horodatage de l'image de la zone de prélèvement acquise avec l'outil d'exérèse.

Un capteur (370) détecte le passage de l'échantillon hors du manchon (600) et fournit un horodatage de ce passage. L'activation du capteur peut, en fonction du résultat de détection délivré par le manchon (600), déclencher une alerte de sécurité (signal sonore ou lumineux) et/ou commander la vanne (230) ). Les deux zones de détection (390, 600) sont distantes d'une distance D₂ et la mesure du temps écoulé entre les deux permet de déterminer la vitesse de déplacement du prélèvement dans le tuyau (200) et caractériser d'éventuelles anomalies par comparaison de la vitesse nominale de déplacement.

Une autre barrière lumineuse (230) fourni également une information sur le temps écoulé entre le passage de l'échantillon dans le manchon et le parcours du tronçon de tuyau de longueur D₁ et de fournir une alerte en cas de dérive par rapport à une durée de référence.

L'installation peut en outre être équipée d'un moyen d'analyse (700) additionnel raccordé au manchon de détection (600), par exemple un spectromètre de masse, ou un compteur de fluorescence.

### Séquençage des prélèvements selon un exemple non limitatif de réalisation

Le chirurgien contrôle l'aspiration des tissus : la tête de l'outil d'exérèse en contact avec les tissus du patient aspire les prélèvements tissulaires qui sont ensuite entraînés à travers le tube (200) jusqu'à l'ensemble de réceptacle (300).

Les prélèvements sont entraînés par cette aspiration à travers le tube (200) et passent dans la zone de référence où le manchon de détection contrôle la présence où l'absence de marqueurs dans l'échantillon le traversant à un instant t, ce qui permet de l'orienter vers un réceptacle de déchets ou un réceptacle d'analyse ultérieur.

Selon un mode de réalisation particulier un capteur de type pressostat détecte la fermeture du trou et donc le démarrage d'une séquence de prélèvement, et l'état de ce capteur est horodaté et enregistré, ainsi éventuellement que l'image de la zone de prélèvement dans la plaie opératoire fournie par la tête de détection (120) , qui est également enregistrée sous forme horodatée.

Les signaux fournis par le manchon (600) sont également enregistrés de manière horodatée.

La resynchronisation des informations prend en compte les temps moyens parcourus par un prélèvement dans le tube (200) dans la longueur D₂ entre l'outil d'exérèse et le manchon (600) et dans la longueur D₁ entre le réceptacle (300) et le manchon (600).

Ces informations permettent de fournir des informations additionnelles pour le tissu prélevé et recueilli à un instant donné. En prévoyant un barillet avec une pluralité de réceptacles, chaque séquence de tissu prélevée est recueillie dans un réceptacle spécifique, associé à un temps de référence permettant éventuellement de lui associer l'image fournie par l'imageur de l'outil d'exérèse ainsi que les informations fournies par le manchon (600).

## Revendications

1. Système de prélèvement de tissus biologiques dans une zone d'intérêt recevant préalablement ou simultanément à l'intervention chirurgicale un biomarqueur, ledit système comportant une tête d'exérèse (100) reliée à un réceptacle (300) des tissus prélevés par un tuyau de transfert (200) par aspiration, **caractérisé en ce qu'**il comporte :
- un manchon (600) de détection entourant une zone de référence dudit tuyau (200) de transfert par aspiration, ledit manchon de détection (600) comportant au moins un capteur sensible audit biomarqueur
- un circuit électronique délivrant un signal électrique représentatif de la présence ou de l'absence de marqueur lors du passage d'un tissu prélevé dans ladite zone de référence.

2. Système de prélèvement de tissus biologiques selon la revendication 1 **caractérisé en ce que** ledit signal électrique commande le transfert du tissu prélevé venant de passer dans ladite zone de référence vers ledit réceptacle de collecte en cas de détection d'un marqueur ou vers un autre réceptacle par défaut.

3. Système de prélèvement de tissus biologiques selon la revendication 1 **caractérisé en ce qu'**il comporte un moyen d'actionnement transitoire de l'aspiration délivrant un signal électrique horodaté, et **en ce que** ledit signal représentatif de la présence d'un marqueur est également horodaté.

4. Système de prélèvement de tissus biologiques selon la revendication 1 **caractérisé en ce que** ledit capteur est formé par un scintillateur organique annulaire (610) qui détecte les particules bêta et les rayons gamma associé à un photodétecteur (630).

5. Système de prélèvement de tissus biologiques selon la revendication précédente **caractérisé en ce que** ledit scintillateur organique annulaire (610) est entouré par un scintillateur gamma (640) inorganique également annulaire, qui ne détecte que les rayons gamma, ledit scintillateur gamma inorganique étant associé à un second photodétecteur (660).

6. Système de prélèvement de tissus biologiques selon la revendication 1 **caractérisé en ce qu'**il comporte une vanne (230) dirigeant les tissus prélevés vers un réceptacle de collecte ou vers un réceptacle de déchet en fonction de l'information délivrée par le manchon de détection (600).

7. Système de prélèvement de tissus biologiques selon la revendication 1 **caractérisé en ce que** ledit réceptacle (300) est constitué par un barillet comportant une pluralité d'unité de collecte.

8. Système de prélèvement de tissus biologiques selon la revendication 1 **caractérisé en ce qu'**il comporte un pressostat détectant l'activation du prélèvement par la tête d'exérèse.

9. Système de prélèvement de tissus biologiques selon la revendication 1 **caractérisé en ce qu'**il comporte une enceinte dont les parois comportent des feuilles de plomb, pour le confinement dudit réceptacle (300).

10. Système de prélèvement de tissus biologiques selon la revendication précédente **caractérisé en ce que** ladite enceinte comporte une pluralité de logement comportant chacun un capteur de rayonnement.

11. Système de prélèvement de tissus biologiques selon la revendication 9 ou 10 **caractérisé en ce que** ladite enceinte comporte en outre un logement pour recevoir une poche à urine.

12. Système de prélèvement de tissus biologiques selon la revendication 1 **caractérisé en ce qu'**il comporte ledit circuit électronique qui commande une alerte de sécurité dans le cas de non-détection de biomarqueur.

13. Système de prélèvement de tissus biologiques selon la revendication 1 **caractérisé en ce qu'**il comporte en outre un moyen d'analyse en temps réel des tissus sur certains aspects histologiques et biochimiques
